# EUROPEAN PATENT APPLICATION

(11) **EP 1 153 618 A1**
(43) Date of publication of application: **14.11.2001**
(21) Application number: 01304251.0
(22) Date of filing: 11.05.2001
(51) Int. Cl.: A61L 2/16, A61L 2/235, A61K 7/48, A61K 31/715, A61P 39/06

(54) **Use of oxidized cellulose as free radical scavenger**

(30) Priority: 12.05.2000 US 569689
(71) Applicant: Johnson & Johnson Medical Ltd., Edinburgh EH2 4NH (GB)
(72) Inventor: Schmidt, Richard, Barnoldswick, Lancashire, BB18 6HJ (GB); Bogan, Declan, Santry, Dublin (IE); Moore, Johnathan, Bradford, West Yorkshire BG3 0HE (GB)
(74) Representative: James, Anthony Christopher W.P.

(57) **Abstract**

The invention provides a method of scavenging free radicals in solution comprising contacting the solution with a composition comprising an oxidized cellulose. Preferably, the solution is a biological fluid and the free radicals are active oxygen radicals. The invention also provides methods of treatment of a medical condition mediated by free radicals comprising administering to a patient an effective amount of a composition comprising an oxidized cellulose. Typical conditions include skin aging and wrinkling, lipodermatosclerosis, inflammation and arthritis. The oxidized cellulose is preferably oxidized regenerated cellulose.

## Description

### Field

The present invention relates to compositions containing oxidized polysaccharides, and the use of such compositions as free radical scavengers in therapeutic and other applications.

### Background

Elevated concentrations of active oxygen species such as hydroxyl radicals (·OH), hydroperoxyl radicals (·OOH) and superoxide anions (·O⁻) and other free radical species such as nitric oxide (NO) are implicated in a number of medical conditions, including wrinkle formation, arthritis, lipodermatosclerosis and inflammation..

The presence of active oxygen radicals is believed to be advantageous in the early stages of wound healing, attracting macrophages into the wound and promoting the respiratory burst. However, their continued presence is thought to be detrimental. They are thought to promote continued inflammation and so delay healing. The presence of reactive oxygen species is also thought to lead to elevated levels of matrix metalloproteinases in wounds. In severe cases the presence of reactive oxygen species can induce tissue necrosis and permanent tissue damage.

In contrast, oxidizing species without unpaired electrons, such as hydrogen peroxide (H₂O₂), are believed to be considerably less harmful. Indeed, Burden, Gill and Rice Evans suggest in Free Radical Research Communications, Vol. 7 (1989), pages 149-159, that low levels of hydrogen peroxide (around 10⁻⁸ to 10⁻⁶ molar) stimulate fibroblast proliferation.

Accordingly, the prevention or treatment of a number of medical conditions may be assisted by the use of antioxidant compositions that reactive specifically with active oxygen species such as the radicals listed above.

US-A-5612321 describes compositions comprising polysaccharides grafted with antioxidants on at least one hydroxyl group of the polysaccharide. The compositions may be used *inter alia* to promote the healing of chronic wounds. Preferably, the polysaccharide is hyaluronic acid and the antioxidant group comprises a phenol group. Similar antioxidant-grafted polysaccharides are described in WO94/13333. Such grafted polysaccharides are relatively expensive to make, and have unknown toxicological properties.

### Summary of the Invention

It is an object of the present invention to provide physiologically acceptable materials having antioxidant properties.

It has now been found that simple oxidation of cellulose confers a reactivity of the resulting oxidized cellulose with free radicals that could renders it useful for the removal of active oxygen species from biological fluids.

In a first aspect, the present invention provides a method of scavenging free radicals in solution comprising contacting the solution with a composition comprising an oxidized cellulose.

Preferably, the solution is a biological fluid. Preferably, the free radicals comprise active oxygen species. The term "scavenging" refers to removing the radicals from solution by adsorption or reaction, or otherwise inactivating the free radicals for example by a single-electron redox process to convert the free radicals into an electron-paired species.

In a second aspect, the present invention provides a method of treatment of a medical condition mediated by free radicals comprising administering to a patient an effective amount of a composition comprising an oxidized cellulose.

Preferably, the composition comprising oxidized cellulose is administered topically. Preferably, the medical condition is selected from the group consisting of wrinkle formation, lipodermatosclerosis, arthritis, inflammation, and combinations thereof.

Preferably, the oxidized cellulose used in the present invention has a free radical activity in the Diphenylpicrylhydrazyl (DPPH) test as defined hereinbelow in procedure 1 of at least 15%.

The term "oxidized cellulose" refers to any material produced by the oxidation of cellulose, for example with dinitrogen tetroxide. Such oxidation converts primary alcohol groups on the saccharide residues to carboxylic acid groups, forming uronic acid residues within the cellulose chain. The oxidation generally does not proceed with complete selectivity, and as a result hydroxyl groups on carbons 2 and 3 are occasionally converted to the keto form. These keto units introduce an alkali label link, which at pH 7 or higher initiates the decomposition of the polymer via formation of a lactone and sugar ring cleavage. As a result, oxidized cellulose is biodegradable and bioabsorbable under phsyiological conditions.

The preferred oxidized cellulose for practical applications is oxidized regenerated cellulose (ORC) prepared by oxidation of a regenerated cellulose, such as rayon. It has been known for some time that ORC has haemostatic properties. ORC has been available as a haemostatic product called SURGICEL (Registered Trade Mark of Johnson & Johnson Medical, Inc.) since 1950. This product is produced by the oxidation of a knitted rayon material.

A modification of porosity, density and knit pattern led to the launch of a second ORC fabric product, INTERCEED (Registered Trade Mark of Johnson & Johnson Medical, Inc.), which was shown to reduce the extent of post-surgical adhesions in abdominal surgery.

WO98/00180 describes the use of ORC and complexes thereof for the treatment of chronic wounds, such as diabetic ulcers. The mechanism of action of the ORC in chronic wound treatment is thought to involve binding and inactivation of matrix metalloproteinase enzymes present in the wound fluid.

WO98/00446 describes the preparation of ORC oligosaccharides by partial hydrolysis of ORC in alkali solution, followed by dialysis and purification. The ORC oligosaccharides are shown to have similar matrix metalloproteinase binding properties to ORC, and are also indicated for the treatment of chronic wounds. The present invention encompasses the use of compositions containing such oxidized cellulose oligosaccharides as the free radical sequestering species.

In the methods according to the present invention, the oxidized cellulose preferably comprises oxidized regenerated cellulose. The ORC may be in the form of fibers or woven or nonwoven fabrics or freeze-dried or solvent-dried sponges.

The oxidized cellulose may be combined with other active substances as well as diluents, supports and excipients. Preferably, at least 10% by weight of the material used in the methods of the present invention consists of oxidized regenerated cellulose, more preferably at least 25% by weight and most preferably at least 40% by weight.

In preferred embodiments of the present invention, the oxidized cellulose is complexed with collagen to form structures of the kind described in WO98/00180 and WO98/00446, the entire contents of which are expressly incorporated herein by reference. For example, the oxidized cellulose may be in the form of milled ORC fibres that are dispersed in a freeze-dried collagen sponge. This provides for certain therapeutic and synergistic effects arising from the complexation with collagen.

Preferably, for certain applications, the material comprising oxidized cellulose used in the present invention is substantially insoluble in water. That is to say, it has a solubility of less than 1g/l in water at 25 °C. Low solubility renders such oxidized cellulose especially suitable for use to remove active oxygen species from biological fluids.

Preferably, the oxidized cellulose used in the present invention is provided in the form of a film, a gel, a web, a woven or nonwoven fabric or a freeze-dried sponge.

### Brief Description of the Drawings

Particular embodiments of the present invention will now be described further, by way of example, with reference to the accompanying drawings, in which:-
Figure 1 shows the results of DPPH assay expressed as optical absorbance at 524 nm for the following test substances: (A) control at t=0; (B) DPPH control at t=final; (C) DPPH + ascorbic acid at t=final; (D) DPPH + FIBRACOL at t=final; (E) DPPH + Collagen/ORC sponge at t=final; (F) DPPH + cellulose paper at t=final; and (G) DPPH + CMC sponge at t=final; and
Figure 2 shows the results of DPPH assay expressed as optical absorbance at 524 nm for DPPH (control) and DPPH + varying amounts of collagen/ORC sponge.

### Detailed Description

### Example 1

A collagen/ORC sponge is prepared in similar fashion to the methods described in WO98/00180. Briefly, purified collagen is suspended in 0.05m acetic acid. Milled ORC powder (milled SURGICEL cloth) is added to the suspension at a weight ratio of 45:55 ORC:collagen to give a total solids concentration of about 0.67% by weight, and the mixture is homogenized. The complex suspension is degassed in a vacuum oven, and is then poured into a tray and frozen to -40°C. The frozen suspension is then freeze-dried and dehydrothermally cross-linked using a programmable freeze-drier with a temperature ramping facility.

### Example 2 (comparative)

A sample of a commercially available collagen/alginate sponge produced by freeze drying a slurry of collagen and alginate substantially as described in US patent 4,614,794 was obtained. The product is commercially available under the registered trade mark FIBRACOL from Johnson & Johnson Medical, Arlington.

### Example 3 (comparative)

A freeze-dried carboxymethylcellulose sponge was prepared by freeze drying a CMC dispersion in water.

### Procedure 1

The ability of the materials to react with and remove oxygen containing free radicals is assessed by the DPPH test described in WO94/13333, the entire content of which is expressly incorporated herein by reference. The test is adapted from that described by M.S. Blois in Nature, Vol. 181, page 1199 (1958), and P.W. Banda, A.E. Sherry and M.S. Blois in Analytical Letters, Vol. 7, page 41 (1974).

Briefly, 40mg of the material under test was suspended in 2.5ml of 0.1 molar pH 7.0 phosphate buffer. A solution was of diphenylpicryl hydroxyl (DPPH) in methanol (10⁻⁴ molar) was added in an amount of 2.5 ml, and the mixture was shaken and stored in the dark at 20°C. The samples were assessed by measurement of their light absorbance at 524 nanometers over 6 hours in comparison with a control, particular attention being paid to figure after 4 hours. The percentage reduction absorbance in comparison with the control after 4 hours gives the DPPH test value, with a reproducibility generally of ±5%.

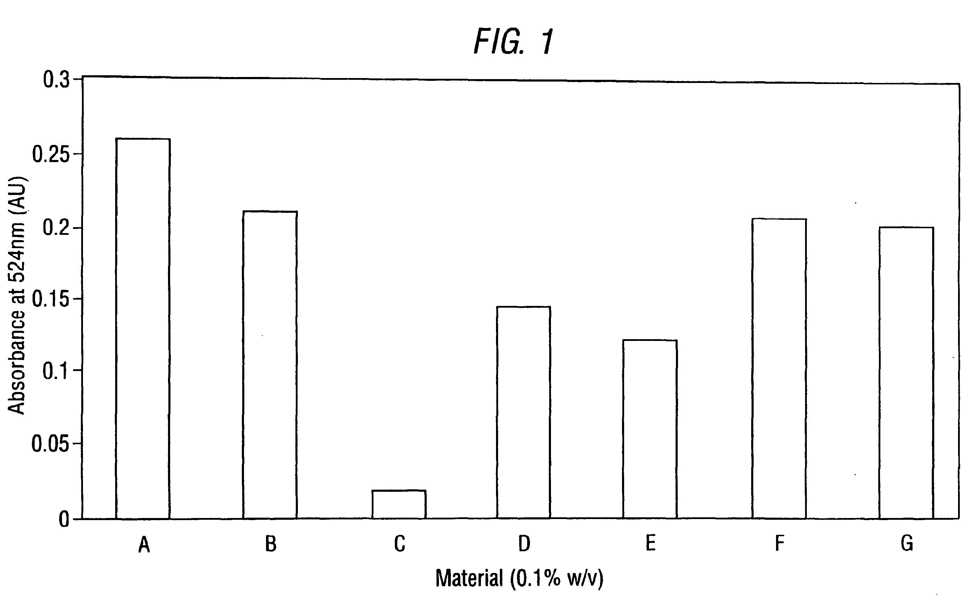

The comparative data in Figure 1 show that DPPH alone exhibits a small decrease in absorbance after 4 hours. However, in the presence of 5mg/ml ascorbic acid, which is a well known free radical scavenger, the DPPH absorbance drops very substantially after 4 hours.

Application of this test to the products of Examples 1 resulted in substantially reduced DPPH test values after 4 hours, as shown in Figure 1 In contrast, the unmodified cellulose filter paper, the collagen/alginate sponge and the carboxymethyl cellulose sponge exhibited much lower DPPH values, generally less than 15%. The activity of the ORC in this test is especially surprising. Polysaccharides and carboxylate groups do not normally act as free radical scavengers. Indeed, the alginate and CMC samples, which contain chemically similar carboxylate groups, do not themselves act as free radical scavengers.

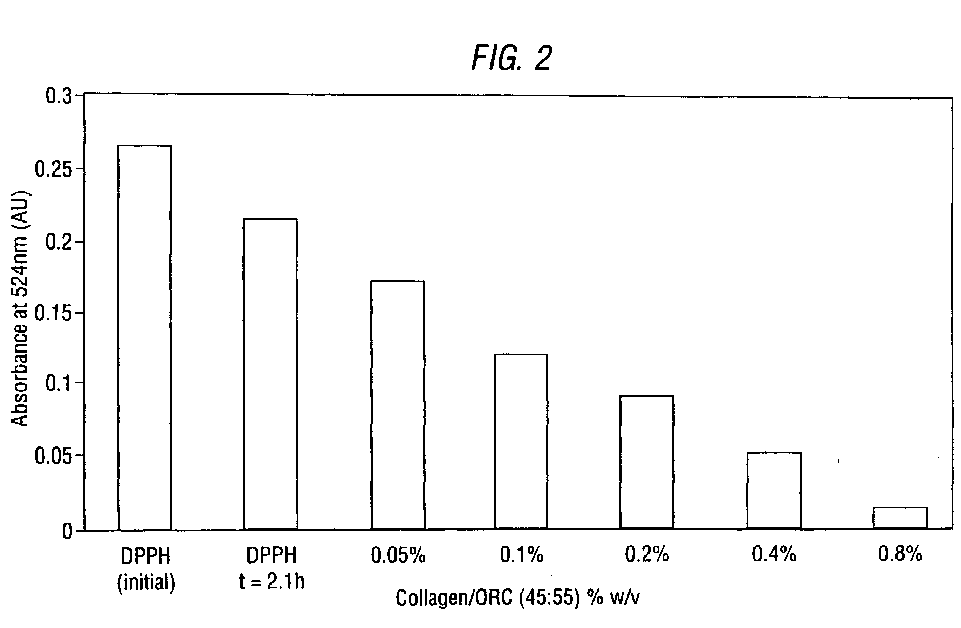

The data shown in Figure 2 confirm a strong dependence for the DPPH scavenging on the amount of collagen/ORC complex of Example 1 that is added to the solution.

The above embodiments have been described by way of example only. Many other embodiments falling within the scope of the accompanying claims will be apparent to the skilled reader.

## Claims

1. A method of scavenging free radicals in solution comprising contacting the solution with a composition comprising an oxidized cellulose.

2. A method according to claim 1, wherein the solution is a biological fluid.

3. A method according to claim 1, wherein the free radicals comprise active oxygen species.

4. A method according to claim 1, wherein the oxidized cellulose consists essentially of oxidized regenerated cellulose (ORC).

5. A method according to claim 4, wherein the composition comprises at least 25% by weight of ORC.

6. A method according to claim 5, wherein the composition is a solid material comprising a freeze-dried sponge of collagen and oxidized regenerated cellulose (ORC).

7. A method according to claim 6, wherein the composition consists essentially of a freeze-dried sponge of collagen and oxidized regenerated cellulose (ORC).

8. A method according to claim 6, wherein the composition comprises an oxidized cellulose oligosaccharide.

9. A method according to claim 1, wherein the composition has an activity in the DPPH assay as herein defined of at least 15%.

10. A method of treatment of a medical condition mediated by free radicals comprising administering to a patient of a clinically effective amount of a composition comprising an oxidized cellulose.

11. A method according to claim 10, wherein the oxidized cellulose consists essentially of oxidized regenerated cellulose (ORC).

12. A method according to claim 11, wherein the composition comprises at least 25% by weight of ORC.

13. A method according to claim 11, wherein the composition is a solid material comprising a freeze-dried sponge of collagen and oxidized regenerated cellulose (ORC).

14. A method according to claim 13, wherein the composition consists essentially of a freeze-dried sponge of collagen and oxidized regenerated cellulose (ORC).

15. A method according to claim 10, wherein the composition comprises an oxidized cellulose oligosaccharide.

16. A method according to claim 10, wherein the composition has an activity in the DPPH assay as herein defined of at least 15%.

17. A method according to claim 10, wherein the medical condition is selected from the list consisting of skin aging and wrinkle formation, lipodermatosclerosis, inflammation and arthritis.
